(19) Europäisches Patentamt
European Patent Office
Office européen des brevets

(11) **EP 4 434 552 B1**

(12) **EUROPEAN PATENT SPECIFICATION**

(45) Date of publication and mention
of the grant of the patent:
**10.12.2025 Bulletin 2025/50**

(21) Application number: **21820654.8**

(22) Date of filing: **17.11.2021**

(51) International Patent Classification (IPC):
*A61L 27/34* (2006.01)   *A61L 27/54* (2006.01)
*A61L 29/08* (2006.01)   *A61L 29/16* (2006.01)
*A61L 31/10* (2006.01)   *A61L 31/16* (2006.01)

(52) Cooperative Patent Classification (CPC):
(C-Sets available)
**A61L 27/54; A61L 27/34; A61L 29/085;
A61L 29/16; A61L 31/10; A61L 31/16;**
A61L 2300/402; A61L 2300/404; A61L 2300/406;
A61L 2300/414; A61L 2300/416; A61L 2300/604;
A61L 2300/608; A61L 2300/61; A61L 2420/08;
(Cont.)

(86) International application number:
**PCT/ES2021/070830**

(87) International publication number:
**WO 2023/089206 (25.05.2023 Gazette 2023/21)**

(54) **A MULTI-LAYERED COATING, A METHOD FOR COATING AN IMPLANT AND AN IMPLANT**

MEHRSCHICHTIGE BESCHICHTUNG, VERFAHREN ZUR BESCHICHTUNG EINES IMPLANTATS UND IMPLANTAT

REVÊTEMENT MULTICOUCHE, PROCÉDÉ DE REVÊTEMENT D'UN IMPLANT ET IMPLANT ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.09.2024 Bulletin 2024/39**

(73) Proprietor: **I+Med S. Coop.**
**01510 Vitoria- Gasteiz Alava (ES)**

(72) Inventors:
• **ANDRADE DEL OLMO, Jon**
**01510 VITORIA- GASTEIZ (ES)**
• **ALONSO CARNICERO, Jose Maria**
**01510 VITORIA- GASTEIZ (ES)**
• **PEREZ GONZALEZ, Raul**
**01510 VITORIA- GASTEIZ (ES)**
• **MUÑOZ MORENTIN, Manuel**
**01510 VITORIA- GASTEIZ (ES)**

(74) Representative: **Galbaian S.Coop.**
**Garaia Parke Teknologikoa
Goiru kalea 1
20500 Arrasate-Mondragón (ES)**

(56) References cited:
**US-A1- 2011 144 229     US-A1- 2014 004 170
US-A1- 2015 071 982**

• **PÉREZ-ÁLVAREZ LEYRE ET AL: "Development
of multiactive antibacterial multilayers of
hyaluronic acid and chitosan onto poly(ethylene
terephthalate)", EUROPEAN POLYMER
JOURNAL, vol. 112, 2019, pages 31 - 37,
XP085611357, ISSN: 0014-3057, DOI: 10.1016/
J.EURPOLYMJ.2018.12.038**

**(Cont. next page)**

- YU YONGLIN ET AL: "Enzyme responsive titanium substrates with antibacterial property and osteo/angio-genic differentiation potentials", COLLOIDS AND SURFACES B: BIOINTERFACES, ELSEVIER AMSTERDAM, NL, vol. 185, 15 October 2019 (2019-10-15), XP085943184, ISSN: 0927-7765, [retrieved on 20191015], DOI: 10.1016/J.COLSURFB.2019.110592
- DEL HOYO-GALLEGO SARA ET AL: "Construction of antibacterial poly(ethylene terephthalate) films via layer by layer assembly of chitosan and hyaluronic acid", CARBOHYDRATE POLYMERS, APPLIED SCIENCE PUBLISHERS , LTD BARKING, GB, vol. 143, 4 February 2016 (2016-02-04), pages 35 - 43, XP029501666, ISSN: 0144-8617, DOI: 10.1016/J.CARBPOL.2016.02.008
- CHUA P. H. ET AL: "Structural stability and bioapplicability assessment of hyaluronic acid-chitosan polyelectrolyte multilayers on titanium substrates", JOURNAL OF BIOMEDICAL MATERIALS RESEARCH PART A, vol. 87A, no. 4, 15 December 2008 (2008-12-15), US, pages 1061 - 1074, XP055948347, ISSN: 1549-3296, DOI: 10.1002/jbm.a.31854
- ALMEIDA ANA CATARINA ET AL: "Bioactive and adhesive properties of multilayered coatings based on catechol-functionalized chitosan/hyaluronic acid and bioactive glass nanoparticles", INTERNATIONAL JOURNAL OF BIOLOGICAL MACROMOLECULES, ELSEVIER BV, NL, vol. 157, 23 April 2020 (2020-04-23), pages 119 - 134, XP086191481, ISSN: 0141-8130, [retrieved on 20200423], DOI: 10.1016/J.IJBIOMAC.2020.04.095
- MARTINS ALESSANDRO F ET AL: "Chitosan/iota-carrageenan and chitosan/pectin polyelectrolyte multilayer scaffolds with antiadhesive and bactericidal properties", APPLIED SURFACE SCIENCE, ELSEVIER, AMSTERDAM, NL, vol. 502, 16 October 2019 (2019-10-16), XP085921963, ISSN: 0169-4332, [retrieved on 20191016], DOI: 10.1016/J.APSUSC.2019.144282

(52) Cooperative Patent Classification (CPC): (Cont.)
A61L 2430/12

C-Sets
A61L 27/34, C08L 5/08;
A61L 29/085, C08L 5/08;
A61L 31/10, C08L 5/08

**Description**

TECHNICAL FIELD

The present invention relates to a polyelectrolyte multilayer coating for implants and methods of its preparation.

PRIOR ART

Implant rejection reactions of implants for permanent or at least moderate-duration residence in human or animal body due to infections caused by the growth of microorganisms on the implant are a big problem. The rejection triggers a reduction of the functionality of the implant and the success of healing in the treatment. Metallic implants usually have rough surfaces. Other kind of implants, for example, dental screws, have also grooves that are needed for the functionality of the implant but promotes the adhesion and proliferation of microorganism in such grooves and rough surfaces.

Applying coatings of biocompatible polymers to the implant for minimizing rejections is known. US2006165962A1 describes a coating system for implants having a metallic main body which comprises a polysaccharide layer made of chitosan and hyaluronic acid for tissue compatibility of the implant.

US2011/144229 A1 discloses biocompatible coatings including a multilayer polyanionic polymeric material and polycationic material using layer by layer technology.

US2015/071982 A1 discloses coatings with a plurality of polymer layers polyanionic polymeric layer and cationic polymer layers for preventing or inhibiting microbial infections.

Document "Development of multiactive antibacterial multilayers of hyaluronic acid and chitosan onto poly(ethylene terephthalate" of Pérez-Alvarez Leyre et al. discloses a method for coating a medical implants coated by a layer by layer of hyaluronic acid and chitosan.

Document "Enzyme responsive titanium substrates with antibacterial property and osteo/angiogenic differentiation potentials" of Yo Yonglin et al. discloses titanium substrates coated by layer by layer polyelectrolyte multilayers of hyaluronic acid and chitosan with antibacterial properties.

Document "Construction of antibacterial poly(ethylene terephthalate) films via layer by layer assembly of chitosan and hyaluronic acid" of Del Hoyo-Gallego Sara et al. discloses a coating method of a poly/ethylene terephthalate by layer by layer of hyaluronic acid and chitosan by a dipping method.

Document "Structural stability and bioapplicability assessment of hyaluronic acid-chitosan polyelectrolyte multilayers on titanium substrates" of Chua P.H. et al. s discloses titanium substrates coated by layer by layer polyelectrolyte multilayers of hyaluronic acid and chitosan by a dipping method.

Document "Bioactive and adhesive properties of multilayered coatings based on catechol.functionalized chitosan/hyalurinc acid and bioactive glass nanoperticles" of Almeida Ana Catarina et al. discloses multilayered coatings based on cathecol-functionalized chitosan/hyaluronic acid used in orthopedic implants.

US2014/004170 A1 discloses a method for preparing a drug-eluting implants comprising the steps of depositing oppositely charged polyelectrolyte layers on at least a portion of the surface of the implant to form a polyelectrolyte multilayer.

DISCLOSURE OF THE INVENTION

The object of the invention is to provide a method for coating an implant with a multi-layered coating, as defined in the claims, and an implant coated totally or partially by the method as defined in the claims.

One aspect of the invention is related to a method for coating an implant with a multi-layered coating, the method being a layer by layer coating method, comprising the following steps:

- a first step of activation of a surface of the implant to be coated by exposing superficial hydroxyl, carboxylate, aldehyde, epoxide, isothiocyanate, amine, halide, or thiol groups;
- a second step of generating the multi-layered coating depositing by spraying interspersed polyelectrolyte layers of a layer that comprises hyaluronic acid of an average molecular weight between 0,01 and 2,70 MDa and a layer that comprises chitosan of an average molecular weight between 0,10 and 1,00 MDa and a deacetylated degree greater than 70% onto the activated surface to be coated, wherein the number of interspersed layers is between 20 and 30, and
- a third step of drying the multi-layered coating,

the resulting multi-layered coating having an external surface with a contact angle between 25° and 35°.

A second aspect of the invention is related to an implant coated totally or partially by the method of the invention for use in the prevention of medical complications consequence of the implantation process such as tissue infections, tissue inflammations and/or poor integration of the implant in the human body.

The method of the invention is simple, easy to apply and a uniform coating on the implant is obtained. In the coated implant, when increasing the number of layers, the surface roughness decreases due to a better uniformity in the coating of the implant obtaining besides a hydrophilic coating.

With the multilayered hydrophilic coating, the adherence of the microorganisms to the surface of the coating, and therefore to the implant, is reduced improving the stability of the coating. In that way, the antibacterial effect of the chitosan or hyaluronic acid increases because due to the antifouling antibacterial property of the multi-layered coating of the invention less microorganisms are adhered, giving rise to a less or no growth or proliferation of unwanted microorganisms on the implant surface.

In this sense, the studies conducted by the inventors have demonstrated the capacity of this biocompatible multi-layered coating for being sterilizable, having a good stability, being loadable with active substances with a sustained release of active substances and maintaining the main morphology of the surface of the implant in a homogeneous way.

These and other advantages and feature of the invention will become evident in view of the drawings and of the detailed description of the invention.

DESCRIPTION OF THE DRAWINGS

Figure 1 shows a graphical depiction of a piece of implant with the multi-layered coating according to one embodiment of the invention.

Figure 2 shows a graphical depiction of a contact angle according to the section A of Figure 1.

Figure 3 shows a comparative SEM microscopy image of coated implants by a dip coating method according to different embodiments of the invention.

Figure 4 shows a comparative SEM microscopy image of coated implants by spraying coating method according to different embodiments of the invention.

Figure 5 shows the contact angle value of the multi-layered coating of several embodiments of the invention.

Figure 6 shows a SEM microscopy image of coated implants according to different embodiments of the invention.

Figure 7 shows a remaining coating weight ratio over time of two embodiments of the invention.

Figure 8 shows active substances release curve over time of a coated implant according to different embodiments of the invention.

Figure 9 shows a general scheme of a method for coating an implant with a multi-layered coating according to one embodiment of the invention.

DETAILED DISCLOSURE OF THE INVENTION

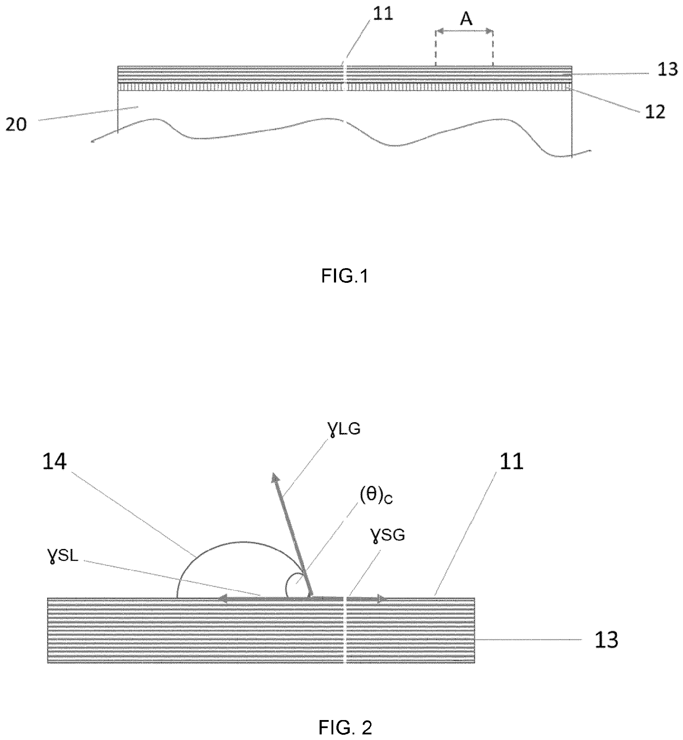

Figure 1 illustrates the multi-layered coating for the surface coating of an implant 20 of the multi-layered coating comprising polyelectrolyte layers wherein the polyelectrolyte layers are interspersed layers of a layer that comprises hyaluronic acid (HA) and a layer that comprises chitosan (CHI) and the multi-layered coating having an external surface 11 with a contact angle between 10° and 50°, preferably between 25° and 35°. The multi-layered coating converts native hydrophobic surfaces to hydrophilic surfaces, gaining antifouling antibacterial property. Besides, considering that bacteria have negatively charged cell walls, those bacteria are repealed from the negatively charged hydrophilic surface of the multi-layered coating by the appearance of electrostatic repulsion forces.

In the context of the invention, as shown in figure 2, the contact angle is the angle conventionally measured through the liquid 14 where a liquid-vapor interface meets a solid surface. It quantifies the wettability of a solid surface by a liquid via the Young equation. The theoretical description arises from the consideration of a thermodynamic equilibrium between the three phases: liquid (L), solid (S), and gas or vapor (G) phase. If the solid-gas interfacial energy is denoted by $\gamma_{SG}$, the solid-liquid interfacial energy by $\gamma_{SL}$. and the liquid-gas interfacial energy by $\gamma_{LG}$ then the contact angle $(\theta)_c$ is determined from these quantities by the Young equation:

$$\gamma_{SG} - \gamma_{SL} - \gamma_{LG} \cos \theta_C = 0$$

In the examples given later, a Dataphysics OCA 15EC Instrument was used for contact angle measurements, and by the way, analyse and quantify water drops contour.

In the context of the invention, the term "hyaluronic acid derivatives" is understood to include all structurally changed reaction products that are obtained when hyaluronic acid undergoes chemical modifications in the active functional groups of its structure (-OH, -COOH, -NH-) through the following reactions: carbodiimidation (preferably, carbodiimide-mediated amidation) esterification, carboxymethylation, alkylation, acylation, thiolation, phosphorylation, amidation, metal coordination, chemical coupling, chemical crosslinking, graft copolymerization, Schiff base, sulfonylation, alkanoylation, sulfation, and sulfonation. Furthermore, the term "hyaluronic acid and hyaluronic acid derivatives" is understood to include all polyelectrolytic salts thereof, for example, sodium, potassium, calcium, and magnesium salts.

In the context of the invention, the term "chitosan derivatives" is understood to include all structurally changed reaction products that are obtained when chitosan undergoes chemical modifications in the active functional groups of its structure (-OH, -NH$_2$ and -NH-) through the following reactions: carbodiimidation (preferably, carbodiimide-mediated amidation), esterification, carboxymethylation, alkylation, acylation, thiolation, phosphorylation, amidation, metal coordination, chemical coupling, chemical crosslinking, graft copolymerization, Schiff base, sulfonylation, alkanoylation, sulfation, and sulfonation.

Regarding the number of layers, in a particular embodiment the number of layers is between 20 and 30. With this number of layers, in addition to achieve the contact angle, the shape or the external appearance of the coated implant is maintained.

Hyaluronic acid has an average molecular weight between 0,01 and 2,70 MDa, preferably, between 2,00 and 2,20 MDa.

Regarding the chitosan, it has an average molecular weight between 0,10 and 1,00 MDa, preferably, between 0,30 and 0,40 MDa, and a deacetylated degree greater than 70%.

In the context of the invention, the degree of deacetylation (DD, %) is defined as the molar fraction of GlcN (d-glucosamine) in the copolymers (chitosan) composed of GlcNAc (N-acetylated glucosamine) and GlcN (d-glucosamine). The degree of deacetylation can be measured by proton nuclear magnetic resonance spectroscopy ($^1$H-NMR) in deuterated water according to the formula

$$DD\ (\%) = (1 - (\frac{I^{\delta H1,90}}{3}/\frac{I^{\delta H3,90-3,00}}{6}))\cdot 100$$

where DD is the deacetylation degree, I$\delta$H1.90 is the intensity of the signal at 1.90 ppm and I$\delta$H3.90-3.00 is the intensity of the signal between 3.90 and 3 ppm. In a preferred embodiment, the degree of deacetylation is greater than 70 %, preferably being between 75 and 85 %.

In a preferred embodiment, the multi-layered coating comprises a first layer 12 of a self-assembled monolayer attached to the surface of the implant, as it is illustrated in figure1. This self-assembled monolayer increases the adherence of HA and CHI and improves or increases the stability of the multi-layered coating during its storage and use. In a preferred embodiment, the self-assembled monolayer is selected from the group consisting of a monolayer based on anchoring groups of silane, phosphonate, phosphate, catechol, carboxylate, amine, alkene, alkyne, 2-hydroxy-1 carboxylate, hydroxamic acid and alkyl iodide, preferably, a monolayer of silane. These monolayers had exposed superficial hydroxyl, carboxylate, aldehyde, epoxide, isothiocyanate, or amine, halide, or thiol groups that reacted with the polyelectrolyte layer.

Regarding the thickness of the multi layered coating, in a preferred embodiment, the thickness is between 10 and 1500 nm, preferably between 470 nm and 1200 nm. With this range of thickness, the coating maintains the shape of the implant, very useful for example in dental implants, wherein some components of the dental implant, such as the abutment and the implant post have grooves for the coupling and it is important to maintain the shape of such grooves.

In a preferred embodiment, the polyelectrolyte layers are loaded with at least one active substance, wherein the active substance is selected from the group of antibiotics, analgesics, anti-inflammatories, antibacterial peptides, antipyretics, growth factors, antidepressants, antihypertensives, antidiabetic, antiepileptic, antiviral and anticancer active substances. This is very useful when a delivery with a sustained release of an active substance in the place where the implant is implanted is needed. The multi-layered coating is able to release the active substance content within the critical period of 24-48 hours for bacterial adhesion and proliferation, but still continue acting as a reservoir of bactericide for a longer-term action. As the exposed layers degrade, the active substance is released from the innermost layers both by diffusion of the active principle through the layers and by the degradation of the outermost layers of the multilayer coating.

In a preferred embodiment, the polyelectrolyte layers are loaded with more than one active substance, preferably the layer that comprises hyaluronic acid (HA) being loaded with one active substance and the layer that comprises chitosan (CHI), being loaded with another active substance. In a preferred embodiment, the layer that comprises hyaluronic acid (HA) is loaded by an active substance selected from antibiotics, analgesics, anti-inflammatories, antibacterial peptides, antipyretics, growth factors, antidepressants, antihypertensives, antidiabetic, antiepileptic, antiviral and anticancer active substances, preferably being loaded by acetyl salicylic acid, cefuroxime, amoxicillin, tetracycline or dexketoprofen or any combination of any of them. In a preferred embodiment, the layer that comprises chitosan (CHI) is loaded by an active substance selected from antibiotics, analgesics, anti-inflammatories, antibacterial peptides, antipyretics, growth factors, antidepressants, antihypertensives, antidiabetic, antiepileptic, antiviral and anticancer active substances, preferably being acetyl salicylic acid, cefuroxime, amoxicillin, tetracycline, dexketoprofeno or any combination of any of them. In a preferred embodiment, both types of interspersed layers are loaded, the layer that comprises hyaluronic acid (HA) being loaded with acetyl salicylic acid, tetracycline and/or dexketoprofen, and the layer that comprises chitosan (CHI) being loaded with amoxicillin and/or tetracycline.

Regarding the use, the multi-layered coating is for use in the prevention of medical complications consequence of the implantation process such as tissue infections, tissue inflammations and/or poor integration of the implant in the human body.

A second aspect of the invention is related to an implant that is coated totally or partially with the method of the invention.

These implants can be dental implants, cochlear implants, orthopaedic implants, cardiac implants, cosmetic implants, zygomatic implants, ocular implants, breast implants, nasal implants, stents, catheters, valves, preferably, dental implants.

In a preferred embodiment, the implant is a metallic implant that comprises metals selected from the group consisting of magnesium metal, magnesium alloy, iron metal, iron alloy, ferrous metal, ferrous alloy, tantalum, cobalt, cobalt alloys, zirconium, zirconium alloys, stainless steel, NiTi alloys, NiTiNOL, gold, silver, platinum, titanium, titanium oxide and titanium alloys, preferably comprising titanium or titanium alloys.

In another embodiment, the implant is a polymeric implant that comprises polymers selected from the group consisting of polytetrafluoroethylene (PTFE), expanded polytetrafluoroethylene (ePTFE), silicone rubber, poly (methyl methacrylate) (PMMA), copoly (lactic-glycolic acid) (PLGA), polyethylene (PE), ultra-high-molecular-weight polyethylene (UHMWPE), polyester urethane urea (PEUU), silicone (polysiloxane), polydimethyl siloxane (PDMS), and carbon nanotubes (CNT).

In another embodiment, the implant is a ceramic implant and comprises materials selected from hydroxyapatite, zirconium oxide (zirconia) and bioactive glasses or a combination of these materials.

The method for coating an implant with a multi-layered coating, the method being a layer by layer coating method, comprises the following steps:

- a first step of activation of a surface of the implant to be coated by exposing superficial hydroxyl, carboxylate, aldehyde, epoxide, isothiocyanate, amine, halide, or thiol groups.
- a second step of generating the multi-layered coating depositing by spraying interspersed polyelectrolyte layers of a layer that comprises hyaluronic acid (HA) of an average molecular weight between 0,01 and 2,70 MDa and a layer that comprises chitosan (CHI) of an average molecular weight between 0,10 and 1,00 MDa and a deacetylated degree greater than 70%, onto the activated surface to be coated, wherein the number of interspersed layers is between 20 and 30,
- a third step of drying the multi-layered coating;

the resulting multi-layered coating having an external surface with a contact angle between 25° and 35°.

To prevent unnecessary repetitions, the features described for the multilayered coating, such as the number of layers, layer thicknesses, type of active substances, combinations of materials are considered to also be described for the respective embodiments of the method of the invention.

In the context of the invention, "by exposing superficial hydroxyl, carboxylate, aldehyde, epoxide, isothiocyanate, amine, halide, or thiol groups" means that due to the activation, the surface of the implant exposes such groups that are going to react with the polyelectrolyte layers to be deposited.

When the activation of the surface of the implant is by exposing amine or halide groups, the first interspersed polyelectrolyte layer to be deposited is the layer that comprises hyaluronic acid (HA).

When the activation of the surface of the implant is by hydroxyl, carboxylate, aldehyde, epoxide, isothiocyanate, or thiol groups, the first interspersed polyelectrolyte layer to be deposited is the layer that comprises chitosan (CHI).

Some non limitative examples of activation method of the surface of the implant, known by the skilled person in the art, are Gas plasma, electrochemistry, acidic wet procedures based on piranha treatments ($HCl:H_2O_2$, $H_2SO_4/H_2O_2$), basic wet procedures based on piranha treatments ($NH_4OH:H_2O_2$), and other basic wet procedures (NaOH, KOH).

In one embodiment, the activation of the surface is made by acidic wet procedures based on piranha treatments ($HCl:H_2O_2$, $H_2SO_4/H_2O_2$). With the acidic wet procedures more active groups are created. In another embodiment, the activation of the surface is made by gas plasma, being the preferred activation procedure for polymeric implants.

In a preferred embodiment, the first step comprises a step of generating or depositing a first layer of self-assembled monolayer on the surface of the implant, preferably, on the surface of the implant previously activated by the exposition of superficial hydroxyl groups, such self-assembled monolayer besides, exposing superficial hydroxyl, carboxylate, aldehyde, epoxide, isothiocyanate, or amine, halide, or thiol groups, preferably amine groups, on which the interspersed polyelectrolyte layers are deposited in second step. The activation and exposition of superficial groups of the self-assembled monolayer is carried out by chemical and physical reactions know by the skilled person in the art.

The method of the invention is carried out by spraying a polyelectrolyte solution of hyaluronic acid or chitosan for generating each polyelectrolyte layer, alternating the polyelectrolyte solutions until the different number of layers are deposited. In a preferred embodiment, the polyelectrolyte concentration in the solution is between 0,1 and 0.2 % (w/w) at pH between 4 and 6, preferably 5. The solvent is selected from the group consisting of acetic acid, formic acid, lactic acid, propionic acid, glycolic acid, tartaric acid, malic acid, citric acid, ascorbic acid, phosphoric acid, and water, preferably acetic acid.

When the second step is by spraying, it is preferred spraying the implants with a spray-angle between 70° and 110°, preferably 90°, the spray-angle being the angle formed by the spray direction and the surface of the material to be coated, and the duration of the spraying step on the area to be coated being preferably between 3 and 5 seconds. The advantage of this duration is that it is enough for assuring the coating of the surface.

In a preferred embodiment, the second step comprises a step of removal of the non-adhered polyelectrolyte between each deposition of the interspersed polyelectrolyte layers. This increases the uniformity of the coating. In a preferred embodiment, the removal step is by spraying the solvent on the last deposited layer or dip coating of the implant in the solvent.

In one embodiment the activation step is by hydroxyl groups and the first polyelectrolyte layer in second step is chitosan.

In another embodiment, the activation is by amine groups and the first polyelectrolyte layer in second step is hyaluronic acid.

In a preferred embodiment, the polyelectrolyte layers are loaded with an active substance. In a preferred embodiment, this loading is achieved by dissolving the active substance in the polyelectrolyte solution in the second step, preferably in a concentration between 0,1-0,2% with respect to the total volume of the solution.

Regarding the third step of drying the multi-layered coating, the drying can be performed by standard methods known by the person skilled in the art. In a preferred embodiment, the drying is by vacuum drying, heat drying, air drying or the combination of all or some of them.

Depending on the use of the coated implant, the implant must be sterilized. In a preferred embodiment the method comprises a fourth step of sterilization. The inventors have demonstrated that the sterilization step does not affect to the integrity and adherence of the multi-layered coating to the implant. The sterilization can be performed by standard methods known by the person skilled in the art, for example, by gamma rays, beta rays and steam sterilization. In a preferred embodiment, the sterilization is by steam sterilization.

Some illustrative examples which clearly show the features and advantages of the invention are described below. However, these examples must not be interpreted as limiting the object of the invention as defined in the claims.

Examples

Materials used for carrying out the examples below:

Screws (EVO_Aurea NP085 screws of 3.5 x 8.5 mm size, made of titanium (Ti) and display a thick layer of Ti oxide on the surface. Titanium grade 5 sheets (Ti6Al4V, 250 mm length x 250 mm width x 0.8 mm thickness) were cut in 1 x 1 cm2 sections by TESOLVIT S. L. Fisherbrand 11201 Series Advanced Ultrasonic Cleaners (115V, 37 kHz, 100% power), acetone (LabKem, 99.6%) and ultrapure water (0.22 $\mu$m Millipak filter) were used to clean the surface of Ti6Al4V samples. Sulfuric acid ($H_2SO_4$, LabKem, 96%) and hydrogen peroxide ($H_2O_2$, LabKem, 30%) were used to activate Ti6Al4V surface by wet chemical procedures. The silane monolayer was created using (3-aminopropyl)triethoxysilane (APTES, Sigma-Aldrich, 98%) and ethanol (EtOH, LabKem, 99.5%). Multilayer construction was carried out using hyaluronic acid (HA, Contipro, 2.109 $\pm$ 0.102 MDa, PDI= 1.003 $\pm$ 0.004) and chitosan (CHI, Sigma-Aldrich, deacetylated degree >75 %, 0.310-0.375 MDa) biopolymers. Acetic acid ($CH_3COOH$, Fischer Chemicals, 99.8%) and sodium hydroxide (NaOH, Panreac, 98%) were employed to prepare acetic/acetate buffer solution. Phosphate buffer saline (PBS, pH =7.4) was also prepared with Fischer Chemicals reagents as is the case of di-sodium hydrogen orthophosphate dodecahydrate ($Na_2HPO_4 \cdot 12H_2O$, 99%), sodium dihydrogen orthophosphate dihydrate ($NaH_2PPO_4 \cdot 2H_2O$, 99%), sodium chloride (NaCl, 99%) and polyvinylidene fluoride filters (PVDF, 0.22 $\mu$m). Loading and release studies from multilayer system was performed using cefuroxime sodium salt (CFX, Sigma-Aldrich) antibiotic, and acetylsalicylic acid (AAS, Acros Organics, 99%) anti-inflammatory agent.

*Example 1: Coating* of a *titanium implant by the multi-layered coating*

Layer-by-layer technique (LbL technique) was applied to coat the screws with hyaluronic acid (HA) and chitosan (CHI) through two coating methods: dip-coating (not covered by the claims) and spray-coating. Both techniques were applied to coat the screws EVO with 6, 10 and 20 multilayers of HA and CHI biopolymers.

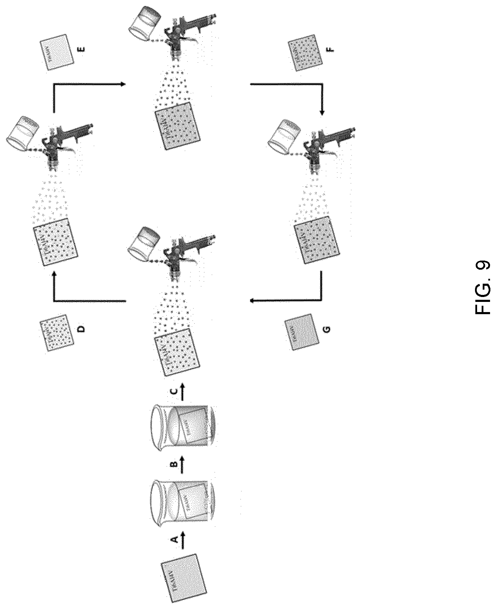

The process of the surface modification of Ti6Al4V samples for the construction of the multilayer coating with HA and CHI is briefly resumed in figure 9.

Step 0: Ti samples are precleaned in ultrapure water, disinfectant soap and acetone.

Step A: Activation of the surface: creation of -OH groups.

Step B: Anchoring of the amino terminated silane monolayer

Step C: Spraying or dip coating of hyaluronic acid (HA) solution.

Step D: Spraying or dip coating of buffer solution.

Step E: Spraying or dip coating of chitosan (CHI) solution.

Step F: Spraying or dip coating of buffer solution.

Step G: Spraying of hyaluronic acid (HA) solution.

repeating n times step C to step G until the desired numbers of layers are deposited. The drying step is not included in the figure.

Hereinafter, by "HA / CHI layers" is meant the number of total layers generated by the interleaved layers of HA and CHI.

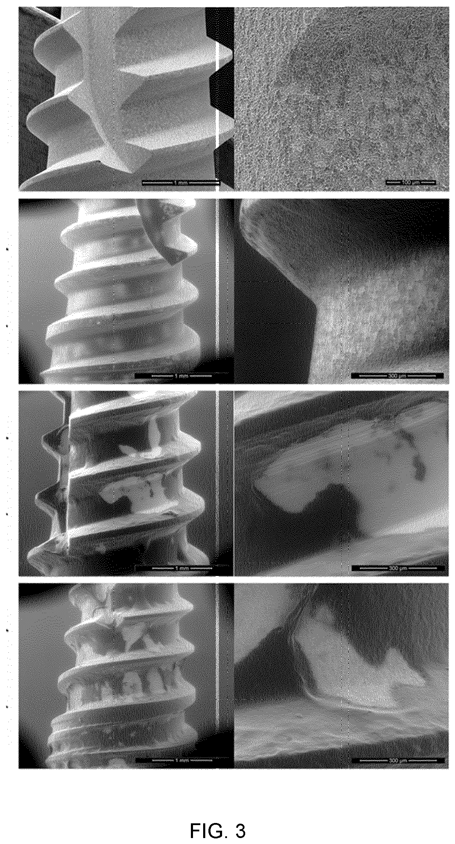

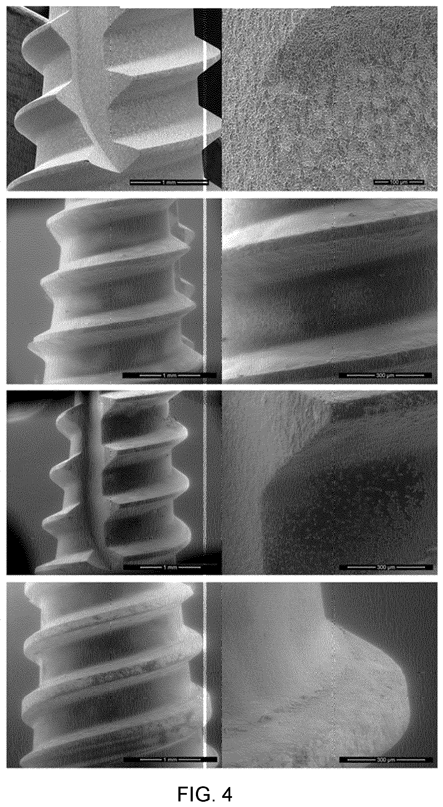

For dip-coating method (not covered by the claims), screws were dipped into HA solution for 5 min, followed with acetic/acetate buffer solution rinsing. After that, the dental implants with the first layer of adsorbed HA were then dipped into a CHI solution for 5 min and followed by the same rinsing process. The alternating polyelectrolyte dipping cycle (hyaluronic acid and chitosan) was performed until the desired number of layers was obtained. Finally, the dip coated dental implants were dried in a vacuum system for 24h at room temperature. Figure 3 shows that the generated coating by dip-coating is distributed on the surface of the screws as the number of deposited multilayers increases. The coating generated by spraying (figure 4) is more homogeneously distributed than the coating produced by dip coating. SEM analysis displays quite a difference in contrast between the coated regions (darker areas) and the uncoated or less coated regions (lighter areas). The first row from the top corresponds to a non-coated sample. The second row corresponds to a screw coated with 6 layers HA/CHI. The third row corresponds to a screw coated with 10 layers HA/CHI and the fourth row corresponds to a screw coated with 20 layers HA/CHI.

LBL assembly using the spray-coating method afforded a more homogenous surface. SEM analysis (figure 4) shows less difference in contrast between the coated regions (darker areas) and the less coated regions (lighter areas) of the screw. Specifically, screws were sprayed with HA solution for 3 seconds, followed with acetic/acetate buffer solution spraying of 1 second to remove the excess of HA not adhered to the surface. Then screws with a layer of adsorbed HA were subsequently sprayed with a CHI solution for 3 seconds and followed with the same process with acetic/acetate buffer solution to remove the excessive non-adhered CHI. The alternating polyelectrolyte spraying cycle was carried out until different number of layers were obtained. Once the desired number of layers has been sprayed onto screws, they dried in a vacuum system for 24h at room temperature.As the number of layers deposited on the surface of the screw increases the quality of the coating improves, as there is less difference in the image contrast between the different areas of the screw which is typical of a homogeneously coated surface. The first row from the top corresponds to a non-coated sample. The second row corresponds to a screw coated with 6 layers HA/CHI. The third row corresponds to a screw coated with 10 layers HA/CHI and the fourth row corresponds to a screw coated with 20 layers HA/CHI.

The spray-system was set up with the following features: the compressed air pressure was adjusted to 0.05-0.1 MPa and the pressure regulator was adjusted to 30-40 cm above the screws. HA and CHI solution are sprayed from the pressure regulator perpendicularly onto the screws with a spray-angle of 70°-110°.

For both the dip-coating and spray-coating procedures employed solutions concentrations were 0.2% (w/w) HA and 0.2% (w/w) CHI (both at pH 5) previously dissolved in 0.5 % (v/v) acetic acid. Acetic/acetate buffer solution (pH 5) was prepared mixing 0.50 M acetic acid and 0.32 M NaOH solutions.

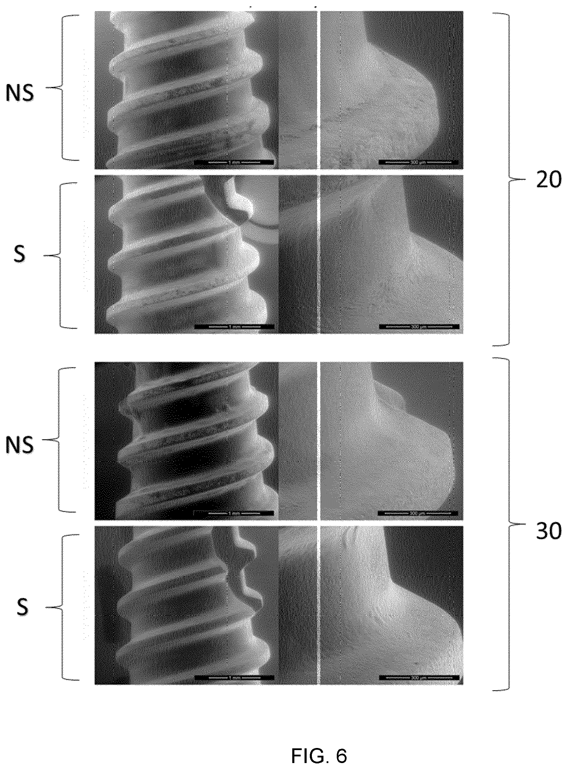

The effect that the sterilization process possesses on the HA/CHI LBL deposited coating by spray-coating was investigated by SEM microscopy (Figure 6- screws covered with 20 HA/CHI layers 20 and 30 HA/CHI layers 30, steam sterilized S and non-sterilized SN), since it could significantly damage the coating. In this case, the steam sterilization method (121 ° C, 15 min) was used.

*Example 2: Surface chemical composition (flat Ti samples)*

Ti samples were coated by the deposition of 0, 10, 20 and 30 layers HA/CHI as described the spraying method in example 1, with and without a monolayer (Silane monolayer).

The chemical composition of the different coated surfaces was determined by X-ray photoelectron spectroscopy (XPS). First, surface composition was analysed in the energy range of 0-800 eV prior and after hydroxylation and 3-aminopropyl)triethoxysilane (APTES) treatment. As it is shown in Table 1, the main elements quantified in all the samples were Ti, C, O, N, Al, and V. A clear increase of the peak appearing at 400 eV, corresponding to nitrogen was observed when pristine and amino treated surfaces were compared, as a result of the insertion of the $NH_2$ groups, demonstrating a successful surface amino functionalization.

Table 1

| Sample | Atomic composition (%) | | | | | | | |
|---|---|---|---|---|---|---|---|---|
| | C$_{1s}$ | O$_{1s}$ | Ti$_{2p}$ | Al$_{2p}$ | V$_{2p\,3/2}$ | N$_{1s}$ | Si$_{2p}$ | Na$_{1s}$ |
| Ti | 28.1 | 42.2 | 24.2 | 3.4 | 0.3 | 1.8 | - | - |
| Ti-Si monolayer | 38.0 | 32.6 | 16.1 | 2.6 | - | 5.6 | 5.1 | - |
| Ti-HA/CHI 10 multilayers without monolayer | 65.9 | 26.9 | - | - | - | 0.7 | - | 6.5 |
| Ti-HA/CHI 10 multilayers with monolayer | 71.4 | 23.0 | - | - | - | 1.5 | - | 4.1 |
| Ti-HA/CHI 20 multilayers without monolayer | 69.1 | 25.5 | - | - | - | 1.6 | - | 3.8 |
| Ti-HA/CHI 20 multilayers with monolayer | 67.8 | 27.2 | - | - | - | 3.3 | - | 1.7 |
| Ti-HA/CHI 30 multilayers without monolayer | 65.2 | 28.0 | - | - | - | 2.7 | - | 4.1 |
| Ti-HA/CHI 30 multilayers with monolayer | 69.6 | 26.0 | - | - | - | 3.9 | - | 0.5 |

The building of the multilayers was confirmed since the signals of the Ti metal substrate (Ti, Al, V), and of the monolayer (Si) decreased to disappear while C clearly increased. In LBL coated samples the N content grows up as the number of deposited layers increases which corroborates the greater HA and CHI quantity adhered to the surface of screws, being Ti-HA/CHI 20 and 30 multilayers with monolayers the screws with the greater HA and CHI quantity in their surface. Moreover, XPS data indicates that the anchor monolayer increases the adherence of HA and CHI since higher N content are measured in these cases.

*Example 3: Surface topography and roughness*

The different topographic effect was observed by Atomic Force Microscopy (AFM) and interferometry techniques when surface modification with 20 and 30 HA/CHI were carried out by the spraying method described in example 1.
The following table shows the Rms and Ra values of pristine and the coated Ti measured by AFM and interferometry.

Table 2

| | AFM (0.75 µm x 0.75 µm area) | | Interferometry (0.5 mm x 0.5 mm area) | |
|---|---|---|---|---|
| Sample | Rms (µm) | Ra (µm) | Rms (µm) | Ra (µm) |
| Ti | 0.54 | 0.43 | 0.64 | 0.50 |
| Ti-HA/CHI 20 multilayers | 0.94 | 0.74 | 1.60 | 1.30 |
| Ti-HA/CHI 30 multilayers | 0.69 | 0.54 | 1.56 | 1.23 |

*Example 4: Surface wettability (flat Ti samples)*

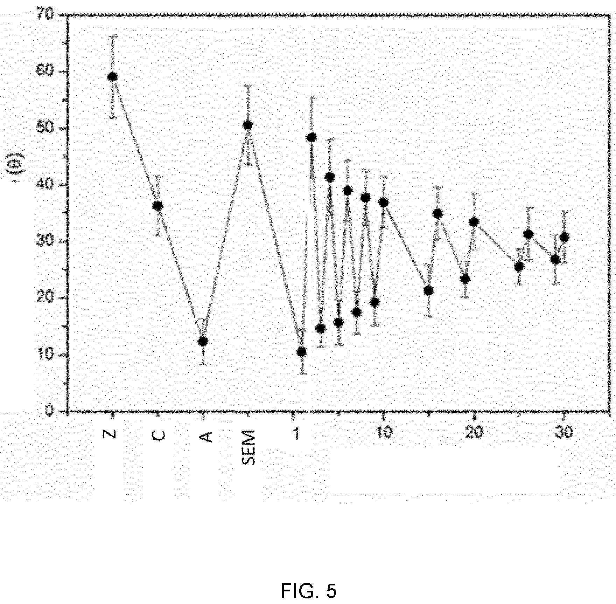

The wettability of unmodified Ti and coated samples according to the spraying method described in example 1 was measured, as observed in figure 5. Surface modification resulted in an increase of hydrophilicity which could be quantified by the measurements of the contact angle. Concretely, the contact angle (θ) of the original Ti samples (Z) sharply decreases when they are cleaned (C) or activated (A) due to the elimination of contaminants and pollutant from the surface of the samples, and the presence of $TiO_2$ and Ti-OH moieties. However, the amino-functionalization by the monolayer of silane (SAM) induces an increase of the contact angle, owning to the introduction of the combination of short alkyl chains

and -NH$_2$ groups.

Following the HA (odd layers) and CHI (even layers) alternative adsorption the contact angle of the coating periodically varied between HA layer and CHI layer. In fact, when few layers were deposited (below 10), the difference between the contact angles of HA and CHI layers reached 25-40°. However, as the number of layers increases, the difference of contact angle between HA and CHI terminated layers decreases due to the interpenetration effect of both layers, until it was almost the same for 29 and 30 multi-layered surfaces.

*Example 5: Thickness of the coating of coated Ti surfaces (flat Ti samples)*

Coated samples according to the spraying method described in example 1, with no loaded active substances was performed by focused ion beam (FIB) and then, the thickness was measured by SEM microscopy.

The thickness of the multilayered coating of 20 and 30 layers HA/CHI could be measured, which were around 470 nm and 1200 nm, respectively.

*Example 6: Stability of HA/CHI multilayer system*

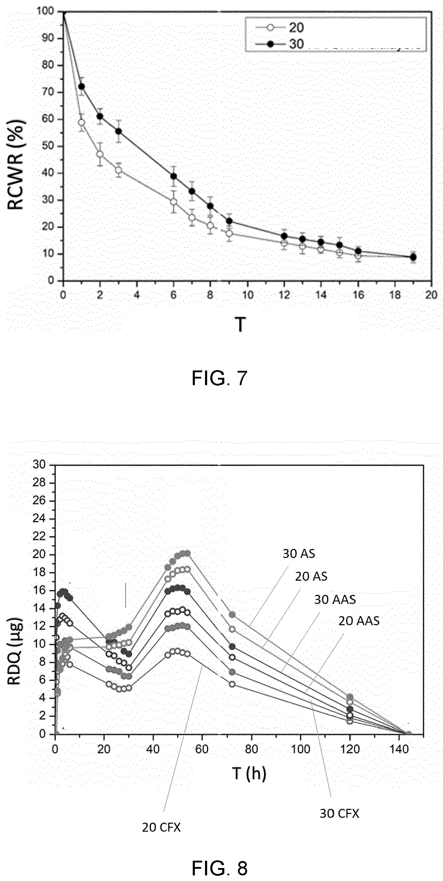

Multilayer stability was studied by immersing Ti-coated samples with 20 and 30 layers HA/CHI, in PBS at 37 °C during the first days after implantation, which is the crucial time for bacterial adhesion and proliferation (24-48 hours). HA/CHI coating degradation was monitored by weight loss ratio (Figure 7: vertical axis corresponds to the remaining coating weight ratio in % (RCWR %); horizontal axis corresponds to time in days (T); 20 corresponds to a 20 layers HA/CHI multilayered coating; 30 corresponds to a 30 layers HA/CHI multilayered coating). The samples were coated according to the spraying method described in example 1. In both cases, a fast initial mass loss could be measured in the first 24h, which can probably correspond to the outer layers not physically adhered. After that, the degradation rate decreases until the ninth day and finally, after the twelfth day, the weight loss remains almost stable. Finally, after 19 days in PBS solution at 37 °C, a 10% coating weight remains already attached to the surface of Ti, proving the stability of the multilayer during the critical period for biofilm formation (24-48h) and posterior period.

*Example 7: Drug loading and release from HA/CHI multilayer system*

Ti samples were covered with the multilayered HA/CHI coating spraying an HA solution with previously dissolved Cefuroxime (CFX) and acetylsalicylic acid (AAS) (0.1% concentration). The procedure to obtain the multilayer coating onto screws with drugs loaded was the same as explained in example 1.

Coated Ti samples were introduced in 75 mL of PBS solution, with gentle agitation at 37 °C and the drug release was measured during several hours by high performance liquid chromatography (HPLC). The release of CFX and AAS in PBS can occur by the diffusion of the drugs through the layers or by the degradation of the surface. As it can be seen in figure 8, the release of the drug occurs especially in the first 24-48 hours. All in all, these results show that prepared multilayers are able to release CFX and AAS content within the critical period of 24-48 hours for bacterial adhesion and proliferation, but still continue acting as a reservoir of bactericide for a longer-term action (6 days).

In figure 8: vertical axis corresponds to the released drug quantity (RDQ); horizontal axis corresponds to time in hours (T); 20 CFX corresponds to a 20 HA/CHI multilayered coating loaded with CFX; 30 CFX corresponds to a 30 HA/CHI multilayered coating loaded with CFX; 20 AS corresponds to a 20 layers HA/CHI multilayered coating loaded with ASS which hydrolyzes to salicylic acid (AS); 30 AS corresponds to a 30 layers HA/CHI multilayered coating loaded with AAS which hydrolyzes to salicylic acid (AS). 20 AAS corresponds to a 20 layers HA/CHI multilayered coating loaded with AAS; 30 AAS corresponds to a 30 layers HA/CHI multilayered coating loaded with AAS.

XPS measurements were performed in order to corroborate the drug release properties and also, the degradation of the multilayer system (Table 3). In fact, in the sixth day of the stability test, the non-presence of S2p and the decrease of N1s corroborates on the one hand, the total release of CFX and AAS from the multilayer system. On the other hand, the stability of the coating is also confirmed, despite start detecting Si$_{2p}$, because other elements from the Ti substrate like Ti, Al and V are not yet detected. N$_{1s}$ and high amounts of C$_{1s}$ are detected as well. The latter confirms the presence of HA and CHI polysaccharide coating. After 19 days of stability test the presence of large amounts of C and N, the non-presence of Ti, Al and V, even having detected Si, continues to demonstrate the stability of the aforementioned HA/CHI coating.

Table 3. Surface atomic compositions (%) of pristine and superficially modified Ti screws with loaded drugs after 6 and 19 stability days in PBS.

| Sample | Atomic composition (%) | | | | | | | | |
|---|---|---|---|---|---|---|---|---|---|
| | C$_{1s}$ | O$_{1s}$ | Ti$_{2p}$ | Al$_{2p}$ | V$_{2p\ 3/2}$ | N$_{1s}$ | Si$_{2p}$ | Na$_{1s}$ | Cl$_{2p}$ | S$_{2p}$ |
| Ti | 28.1 | 42.2 | 24.2 | 3.4 | 0.3 | 1.8 | - | - | - | - |

(continued)

| Sample | Atomic composition (%) | | | | | | | | | |
|---|---|---|---|---|---|---|---|---|---|---|
| | $C_{1s}$ | $O_{1s}$ | $Ti_{2p}$ | $Al_{2p}$ | $V_{2p\,3/2}$ | $N_{1s}$ | $Si_{2p}$ | $Na_{1s}$ | $Cl_{2p}$ | $S_{2p}$ |
| Ti-Si monolayer | 38.0 | 32.6 | 16.1 | 2.6 | - | 5.6 | 5.1 | - | - | - |
| Ti-HA/CHI 20 multilayers with loaded drugs (t=0) | 72.2 | 20.3 | - | - | - | 5.2 | - | 1.4 | - | 0.7 |
| Ti-HA/CHI 20 multilayers with loaded drugs (t=6 days) | 67.2 | 19.2 | - | - | - | 3.3 | 6.2 | 0.7 | 3.4 | - |
| Ti-HA/CHI 20 multilayers with loaded drugs (t=19 days) | 65.7 | 22.7 | - | - | - | 4.8 | 6.8 | - | - | - |
| Ti-HA/CHI 30 multilayers with loaded drugs (t=0) | 72.4 | 19.6 | - | - | - | 5.6 | - | 1.5 | | 0.9 |
| Ti-HA/CHI 30 multilayers with loaded drugs (t=6 days) | 63.1 | 18.6 | - | - | - | 3.5 | 3.6 | 2.3 | 8.9 | - |
| Ti-HA/CHI 30 multilayers with loaded drugs (t=19 days) | 67.9 | 22.7 | - | - | - | 4.5 | 4.9 | - | - | - |

*Example 8: Antibacterial studies*

The antibacterial activity of coated samples according to the method described in example 1, some with active substances (Ti-HA/CHI with drugs) and other without active substances (Ti-HA/CHI without drugs), was quantitatively determined, and qualitatively observed according to ISO 22196:2011 - "Measurement of antibacterial activity on plastics and other non-porous surfaces". 24 h was the selected time since it is well-known to be most critical period to acquire dangerous and harmful bacterial infections.

Table 4: Quantitative antibacterial activity results (CFU, bacterial reduction and $\log_{10}$ reduction (R)) of Ti6AI4V control, Ti-HA/CHI without drugs, and Ti-HA/CHI with drugs samples against *Staphylococcus aureus* and *Escherichia coli* bacteria strains at 24 h.

| Sample | Colony forming units (CFU) | | Bacterial reduction (% death) | | log10 reduction (R) | |
|---|---|---|---|---|---|---|
| | *S. aureus* | *E. coli* | *S. aureus* | *E. coli* | *S. aureus* | *E. coli* |
| Ti6AI4V control | $1.44 \cdot 10^5 \pm 2.02 \cdot 10^5$ | $1.55 \cdot 10^5 \pm 3.48 \cdot 10^4$ | - | - | - | - |
| Ti-HA/CHI without drugs | < 200 | 303 ± 70 | 99.86 | 99.80 | 2.86 | 2.71 |
| Ti-HA/CHI with drugs | 1200 ± 600 | < 200 | 99.17 | 99.99 | 2.08 | 3.89 |

*\* "< 200" value is indicated when there is an absence of CFUs due to the confidence interval of the method.*

As stated by ISO 22196:2011 standard, a material is considered to have an effective antibacterial activity when log10 reduction or antibacterial activity values (R) are greater than 2. Taking this into account, as it can be observed in the Table 4 above, it can be said that HA/CHI coating onto Ti6Al4V possess antibacterial behaviour against gram-positive and gram-negative bacteria strains with antibacterial activity values greater than 2 (R= 2.86 for S. aureus and R= 2.71 for *E. coli*). Then, after the loading of CFX and AAS drugs between HA and CHI layers, although the antibacterial activity was not improved against S. aureus (R= 2.08), the number of viable bacteria (CFU) even decreases more significantly and effectively against E. *coli* with the greater antibacterial activity value (R= 3.89).

## Claims

1. A method for coating an implant with a multi-layered coating, the method being a layer by layer coating method, comprising the following steps:

- a first step of activation of a surface of the implant to be coated by superficial hydroxyl carboxylate, aldehyde, epoxide, isothiocyanate, amine, halide, or thiol groups,
- a second step of generating the multi-layered coating (13) depositing by spraying interspersed polyelectrolyte layers of a layer that comprises hyaluronic acid (HA) of an average molecular weight between 0,01 and 2,70 MDa and a layer that comprises chitosan (CHI) of an average molecular weight between 0,10 and 1,00 MDa and a deacetylated degree greater than 70%, onto the activated surface to be coated, wherein the number of interspersed layers is between 20 and 30, and
- a third step of drying the multi-layered coating,
the resulting multi-layered coating having an external surface (11) with a contact angle between 25° and 35°.

2. The method according to claim 2, wherein the average molecular weight of hyaluronic acid is between 2,00 and 2,20 MDa.

3. The method according to claim 1 or 2, wherein the average molecular weight of chitosan is between 0,30 and 0,40 MDa.

4. The method according to any of the preceding claims, wherein the second step is carried out by spraying, with a spray-angle between 70° and 110°, preferably 90°.

5. The method according to any of the preceding claims, wherein the duration of the spraying step on the area to be coated is between 3 and 5 seconds.

6. The method according to any of the preceding claims, wherein the first step comprises a step of generating a first layer of self -assembled monolayer on the surface of the implant, such self- assembled monolayer exposing superficial hydroxyl, carboxylate, aldehyde, epoxide, isothiocyanate, or amine, halide, or thiol groups, preferably amine groups, on which the interspersed polyelectrolyte layers are deposited in second step.

7. The method according to any of the preceding claims, wherein the second step comprises a step of removal of the non-adhered polyelectrolyte between each deposition of the interspersed polyelectrolyte layer.

8. The method according to any of the preceding claims, wherein the polyelectrolyte layers are loaded with at least one active substance, the active substance being preferably selected from the group of antibiotics, analgesics, anti-inflammatories, antibacterial peptides, antipyretics, growth factors, antidepressants, antihypertensives, antidiabetic, antiepileptic, antiviral and anticancer active substances.

9. The method according to any of the preceding claims, wherein the activation is by hydroxyl groups and the first interspersed polyelectrolyte layer in second step is chitosan (CHI).

10. The method according to any of the preceding claims, wherein the activation is by amine groups and the first interspersed polyelectrolyte layer in second step is hyaluronic acid (HA).

11. The method according to any of the preceding claims, wherein the third step of drying is by vacuum drying, heat drying, air drying or the combination of any of them.

12. The method according to any of the preceding claims, wherein the method comprises a fourth step of sterilization, preferably, steam sterilization.

13. The method according to any of the preceding claims, wherein the thickness of the multi-layered coating is between 10 nm and 5000 nm, preferably between 470 nm and 1200 nm.

14. The method according to any of the preceding claims, wherein the implant is a metallic implant or a polymeric implant.

15. An implant coated totally or partially by the method according to any of the preceding claims for use in the prevention of medical complications consequence of the implantation process such as tissue infections, tissue inflammations and/or poor integration of the implant in the human body.

**Patentansprüche**

1. Verfahren zum Beschichten eines Implantats mit einer mehrschichtigen Beschichtung, wobei das Verfahren ein Schicht-für-Schicht-Beschichtungsverfahren ist, umfassend die folgenden Schritte:

    - einen ersten Schritt der Aktivierung einer Oberfläche des zu beschichtenden Implantats durch oberflächliche Hydroxyl-, Carboxylat-, Aldehyd-, Epoxid-, Isothiocyanat-, Amin-, Halogenid- oder Thiolgruppen,
    - einen zweiten Schritt des Generierens der mehrschichtigen Beschichtung (13), wobei durch Sprühen eingelagerte Polyelektrolytschichten einer Schicht, die Hyaluronsäure (HA) mit einem durchschnittlichen Molekulargewicht zwischen 0,01 und 2,70 MDa umfasst, und einer Schicht, die Chitosan (CHI) mit einem durchschnittlichen Molekulargewicht zwischen 0,10 und 1,00 MDa und einem Deacetylierungsgrad von mehr als 70 % umfasst, auf die zu beschichtende aktivierte Oberfläche aufgebracht werden, wobei die Anzahl der eingelagerten Schichten zwischen 20 und 30 liegt, und
    - einen dritten Schritt des Trocknens der mehrschichtigen Beschichtung,

    wobei die resultierende mehrschichtige Beschichtung eine äußere Oberfläche (11) mit einem Kontaktwinkel zwischen 25° und 35° aufweist.

2. Verfahren nach Anspruch 2, wobei das durchschnittliche Molekulargewicht der Hyaluronsäure zwischen 2,00 und 2,20 MDa liegt.

3. Verfahren nach Anspruch 1 oder 2, wobei das durchschnittliche Molekulargewicht des Chitosans zwischen 0,30 und 0,40 MDa liegt.

4. Verfahren nach einem der vorhergehenden Ansprüche, wobei der zweite Schritt durch Sprühen mit einem Sprühwinkel zwischen 70° und 110°, vorzugsweise 90°, durchgeführt wird.

5. Verfahren nach einem der vorhergehenden Ansprüche, wobei die Dauer des Sprühschritts auf der zu beschichtenden Fläche zwischen 3 und 5 Sekunden liegt.

6. Verfahren nach einem der vorhergehenden Ansprüche, wobei der erste Schritt einen Schritt des Generierens einer ersten Schicht einer selbst-assemblierten Monoschicht auf der Oberfläche des Implantats umfasst, wobei eine solche selbstassemblierte Monoschicht oberflächliche Hydroxyl-, Carboxylat-, Aldehyd-, Epoxid-, Isothiocyanat- oder Amin-, Halogenid- oder Thiolgruppen, vorzugsweise Amingruppen, freilegt, auf denen die eingelagerten Polyelektrolytschichten im zweiten Schritt aufgebracht werden.

7. Verfahren nach einem der vorhergehenden Ansprüche, wobei der zweite Schritt einen Schritt der Entfernung des nicht haftenden Polyelektrolyten zwischen jedem Aufbringen der eingelagerten Polyelektrolytschicht umfasst.

8. Verfahren nach einem der vorhergehenden Ansprüche, wobei die Polyelektrolytschichten mit mindestens einem Wirkstoff beladen sind, wobei der Wirkstoff vorzugsweise aus der Gruppe der Antibiotika, Analgetika, Entzündungshemmer, antibakteriellen Peptide, Antipyretika, Wachstumsfaktoren, Antidepressiva, Antihypertensiva, Antidiabetika, Antiepileptika, antiviralen und krebshemmenden Wirkstoffe ausgewählt ist.

9. Verfahren nach einem der vorhergehenden Ansprüche, wobei die Aktivierung durch Hydroxylgruppen erfolgt und die erste eingelagerte Polyelektrolytschicht im zweiten Schritt Chitosan (CHI) ist.

10. Verfahren nach einem der vorhergehenden Ansprüche, wobei die Aktivierung durch Amingruppen erfolgt und die erste eingelagerte Polyelektrolytschicht im zweiten Schritt Hyaluronsäure (HA) ist.

11. Verfahren nach einem der vorhergehenden Ansprüche, wobei der dritte Schritt des Trocknens durch Vakuumtrocknen, Wärmetrocknen, Lufttrocknen oder eine Kombination davon erfolgt.

12. Verfahren nach einem der vorhergehenden Ansprüche, wobei das Verfahren einen vierten Schritt der Sterilisation, vorzugsweise eine Dampfsterilisation, umfasst.

13. Verfahren nach einem der vorhergehenden Ansprüche, wobei die Stärke der mehrschichtigen Beschichtung zwischen 10 nm und 5000 nm, vorzugsweise zwischen 470 nm und 1200 nm liegt.

14. Verfahren nach einem der vorhergehenden Ansprüche, wobei das Implantat ein metallisches Implantat oder ein polymeres Implantat ist.

15. Implantat, das ganz oder teilweise durch das Verfahren nach einem der vorhergehenden Ansprüche beschichtet ist, zur Verwendung bei der Vorbeugung medizinischer Folgekomplikationen des Implantationsprozesses, wie z.B. Gewebeinfektionen, Gewebeentzündungen und/oder mangelhafte Integration des Implantats in den menschlichen Körper.

**Revendications**

1. Procédé de revêtement d'un implant avec un revêtement multicouche, le procédé étant un procédé de revêtement couche par couche procédé, comprenant les étapes suivantes :

   - une première étape d'activation d'une surface de l'implant à revêtir par des groupements superficiels d'hydroxyl-carboxylate, aldéhyde, époxyde, isothiocyanate, amine, halogénure ou thiol,
   - une deuxième étape de génération du revêtement multicouche (13) en déposant par pulvérisation des couches de polyélectrolyte intercalées d'une couche qui comprend de l'acide hyaluronique (HA) ayant un poids moléculaire moyen compris entre 0,01 et 2,70 MDa et une couche comprenant du chitosane (CHI) ayant un poids moléculaire moyen compris entre 0,10 et 1,00 MDa et un degré désacétylé supérieur à 70%, sur la surface activée à revêtir, dans lequel le nombre de couches intercalées est compris entre 20 et 30, et
   - une troisième étape de séchage du revêtement multicouche,

   le revêtement multicouche résultant ayant une surface externe (11) avec un angle de contact compris entre 25° et 35°.

2. Procédé selon la revendication 2, dans lequel le poids moléculaire moyen de l'acide hyaluronique est compris entre 2,00 et 2,20 MDa.

3. Procédé selon la revendication 1 ou 2, dans lequel le poids moléculaire moyen du chitosane est compris entre 0,30 et 0,40 MDa.

4. Procédé selon l'une quelconque des revendications précédentes, dans lequel la deuxième étape est mise en œuvre par pulvérisation, avec un angle de pulvérisation compris entre 70° et 110°, de préférence 90°.

5. Procédé selon l'une quelconque des revendications précédentes, dans lequel la durée de l'étape de pulvérisation sur la zone à revêtir est comprise entre 3 et 5 secondes.

6. Procédé selon l'une quelconque des revendications précédentes, dans lequel la première étape comprend une étape de génération d'une première couche de monocouche auto-assemblée sur la surface de l'implant, telle monocouche auto-assemblée exposant des groupements superficiels d'hydroxyle, carboxylate, aldéhyde, époxyde, isothiocyanate, ou amine, halogénure ou thiol, de préférence des groupements amine, sur laquelle sont déposées les couches de polyélectrolyte intercalées dans la deuxième étape.

7. Procédé selon l'une quelconque des revendications précédentes, dans lequel la deuxième étape comprend une étape d'élimination du polyélectrolyte non collé entre chaque déposition de la couche de polyélectrolyte intercalée.

8. Procédé selon l'une quelconque des revendications précédentes, dans lequel les couches de polyélectrolyte sont chargées avec au moins une substance active, la substance active étant choisie de préférence dans le groupe des substances actives antibiotiques, analgésiques, anti-inflammatoires, peptides antibactériens, antipyrétiques, facteurs de croissance, antidépressives, antihypertenseurs, antidiabétiques, antiépileptiques, antivirales et anticancéreuses.

9. Procédé selon l'une quelconque des revendications précédentes, dans lequel l'activation est réalisée par des groupements hydroxyle et la première couche de polyélectrolyte intercalée dans la deuxième étape est du chitosane (CHI).

10. Procédé selon l'une quelconque des revendications précédentes, dans lequel l'activation est réalisée par des groupements amine et la première couche de polyélectrolyte intercalée dans la deuxième étape est de l'acide hyaluronique (HA).

11. Procédé selon l'une quelconque des revendications précédentes, dans lequel la troisième étape de séchage est réalisée par séchage sous vide, séchage par chaleur, séchage à l'air ou la combinaison de l'un quelconque de ceux-ci.

12. Procédé selon l'une quelconque des revendications précédentes, dans lequel le procédé comprend une quatrième étape de stérilisation, de préférence stérilisation à la vapeur.

13. Procédé selon l'une quelconque des revendications précédentes, dans lequel l'épaisseur du revêtement multicouche est comprise entre 10 nm et 5000 nm, de préférence entre 470 nm et 1200 nm.

14. Procédé selon l'une quelconque des revendications précédentes, dans lequel l'implant est un implant métallique ou un implant polymérique.

15. Implant revêtu totalement ou partiellement par le procédé selon l'une quelconque des revendications précédentes pour son utilisation dans la prévention de complications médicales résultant du procédé d'implantation telles que des infections tissulaires, des inflammations tissulaires et/ou une mauvaise intégration de l'implant dans le corps humain.

FIG.1

FIG. 2

FIG. 3

FIG. 4

FIG. 5

FIG. 6

FIG. 7

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006165962 A1
- US 2011144229 A1
- US 2015071982 A1
- US 2014004170 A1

**Non-patent literature cited in the description**

- **PÉREZ-ALVAREZ LEYRE**. *Development of multi-active antibacterial multilayers of hyaluronic acid and chitosan onto poly(ethylene terephthalate*
- **YO YONGLIN**. *Enzyme responsive titanium substrates with antibacterial property and osteo/angiogenic differentiation potentials*
- **DEL HOYO-GALLEGO SARA**. *Construction of antibacterial poly(ethylene terephthalate) films via layer by layer assembly of chitosan and hyaluronic acid*
- **CHUA P.H.** *Structural stability and bioapplicability assessment of hyaluronic acid-chitosan polyelectrolyte multilayers on titanium substrates*
- **ALMEIDA ANA CATARINA**. *Bioactive and adhesive properties of multilayered coatings based on catechol.functionalized chitosan/hyalurinc acid and bioactive glass nanoperticles*